# EUROPEAN PATENT SPECIFICATION

(11) **EP 1 448 500 B1**
(45) Date of publication and mention of the grant of the patent: **16.11.2011**
(21) Application number: 02765931.7
(22) Date of filing: 31.07.2002
(51) Int. Cl.: C07C 1/20, C07C 1/24

(54) **OXYGENATED HYDROCARBON COMPOSITIONS AND METHOD FOR RECOVERING THE COMPOSITIONS**
SAUERSTOFFHALTIGE KOHLENWASSERSTOFFZUSAMMENSETZUNGEN UND VERFAHREN ZUR GEWINNUNG DIESER ZUSAMMENSETZUNGEN
COMPOSITIONS HYDROCARBONEES OXYGENEES ET PROCEDE DE RECUPERATION DE CES COMPOSITIONS

(30) Priority: 30.11.2001 US 998512
(43) Date of publication of application: 25.08.2004
(73) Proprietor: ExxonMobil Chemical Patents Inc., Baytown, TX 77520-2101 (US)
(72) Inventor: CHENG, Minquan, Evansville, IN 47712 (US); NICOLETTI, Michael, P., Houston, TX 77059 (US)
(74) Representative: Mareschal, Anne
(86) International application number: PCT/US2002/024577
(87) International publication number: WO 2003/048085

(56) References cited:
- EP-A- 0 065 112
- US-A- 6 072 095
- US-A- 6 121 504
- KIRK-OTHMER: "Encyclopedia of chemical Technologiy" 1981 , JOHN WILEY & SONS , NEW YORK XP002224449 page 15, column 3

## Description

### FIELD OF THE INVENTION

This invention is directed to a method of recovering oxygenates from an olefin production process and the oxygenate compositions produced. More specifically, this invention is directed to recovering oxygenate from the product stream of an oxygenate to olefin process.

### BACKGROUND OF THE INVENTION

Olefins, in particular light olefins such as ethylene, propylene and butylene, are conventionally produced from petroleum feedstocks by either catalytic or steam cracking. Oxygenates, however are becoming an alternative feedstock for making light olefins. Particularly promising oxygenate feedstocks are alcohols, such as methanol and ethanol, dimethyl ether, methyl ethyl either, diethyl either, dimethyl carbonate, and methyl formate. Many of these oxygenates can be produced from a variety of sources including synthesis gas derived from natural gas; petroleum liquids; carbonaceous materials, including coal; recycled plastics; municipal wastes; or any appropriate organic material. Because of the wide variety of sources, alcohol, alcohol derivatives, and other oxygenates have promise as an economical, non-petroleum source for light olefin production.

One way of producing olefins is by the catalytic conversion of methanol using a silicoaluminophosphate (SAPO) molecular sieve catalyst. For example, U.S. Patent No. 4,499,327 discloses making olefins from methanol using any of a variety of SAPO molecular sieve catalysts. The Kaiser process is carried out at a temperature between 300°C and 500°C, a pressure between 0.1 atmosphere to 100 atmospheres, and a weight hourly space velocity (WHSV) of between 0.1 and 40 hr⁻¹

Reducing the percent conversion to olefin in the oxygenate reaction process can result in a better yield of olefin products such as ethylene and propylene, and a decrease in unwanted by-products. For example, U.S. Patent No. 6,137,022 discloses that less than complete oxygenate conversion is more than offset by the significant decrease in unwanted by-products. This patent further discloses a process for removing oxygenated hydrocarbon compounds from the olefin products. EP0065112 discloses a process for producing olefins. US6,072,504 discloses a process for producing a tertiary olefin. US6,121,504 discloses a process for converting oxygenates to olefins.

Although the concentration of oxygenated hydrocarbon compounds found in olefin products made from an oxygenate reaction process are generally quite low, the total amount of oxygenated hydrocarbon produced can be substantial. This is particularly true for large scale olefin reaction processes. It would, therefore, be advantageous to more effectively recover oxygenated hydrocarbon compounds found in the olefin product stream of an oxygenate reaction. The recovered oxygenated hydrocarbon compounds can be recycled or sent to alternative processing, rather than discarded, for example, in a wastewater stream.

### SUMMARY OF THE INVENTION

According to the invention there is provided a process as defined in any one of the accompanying claims. This invention provides an effective method for recovering oxygenated hydrocarbon compounds found in the olefin product stream of an oxygenate reaction process. The recovered compounds are suitable for recycling to the oxygenate reaction process or they can be used for alternative processing.

In one embodiment, the method comprises contacting an oxygenate with a molecular sieve catalyst to form an olefin composition, wherein the olefin composition comprises olefin, water and oxygenated hydrocarbon. The olefin composition is cooled to form a liquid water containing stream and an olefin containing vapor stream, wherein the water containing stream comprises at least 1 wt % oxygenated hydrocarbon. The water containing stream is separated from the vapor stream, and the vapor stream is compressed. An olefin product stream and an oxygenated hydrocarbon containing stream are separated from the compressed vapor stream, and the oxygenated hydrocarbon containing stream separated from the compressed vapor stream is combined with the cooled, liquid water containing stream. An oxygenated hydrocarbon product is then recovered from the combined water containing stream and liquid oxygenated hydrocarbon containing stream.

In another embodiment, the olefin product is further separated into ethylene and propylene containing streams. The ethylene containing stream is desirably polymerized, as well as the propylene containing stream.

In yet another embodiment, the vapor stream is compressed at a pressure of at least 30 psia (207 kPa). Desirably, the oxygenated hydrocarbon product contains not greater than 50 wt % water.

The invention further provides a hydrocarbon composition comprising not greater than 50 wt % water,
at least 25 wt % (a), wherein:
(a) is a compound of Formula I

   R-OH Formula I

   wherein R is C₁ to C₅ alkyl;
   and from 10 ppm by weight to 10 wt % of at least two compounds selected from the group consisting of (b), (c), (d), or a combination thereof, wherein:
(b) is a compound of Formula II

   R₁-O-R₂ Formula II

   wherein R₁ is C₁ to C₄ alkyl and R₂ is C₁ to C₄ alkyl, wherein R₁ may be the same as or different than R₂;
(c) is a compound of Formula III 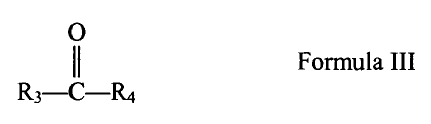 wherein R₃ is C₁ to C₃ alkyl and R₄ is C₁ to C₃ alkyl, wherein R₃ may be the same as or different than R₄; and
(d) is a compound of Formula IV 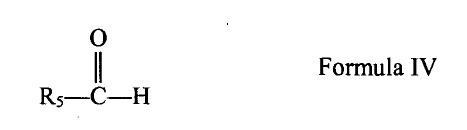 wherein R₅ is C₁ to C₅ alkyl.

Optionally, the compositions of this invention further comprise from 5 ppm by weight to 5 wt % (e), (f), or a combination thereof, wherein:
(e) is a compound of Formula V 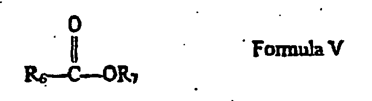 wherein R₆ is C₁ to C₃ alkyl and R₇ is C₁ to C₃ alkyl, wherein R₆ may be the same as or different than R₇: and
(f) is a compound of Formula VI 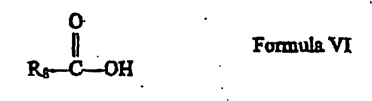 wherein R₈ is C₁ to C₅ alkyl.

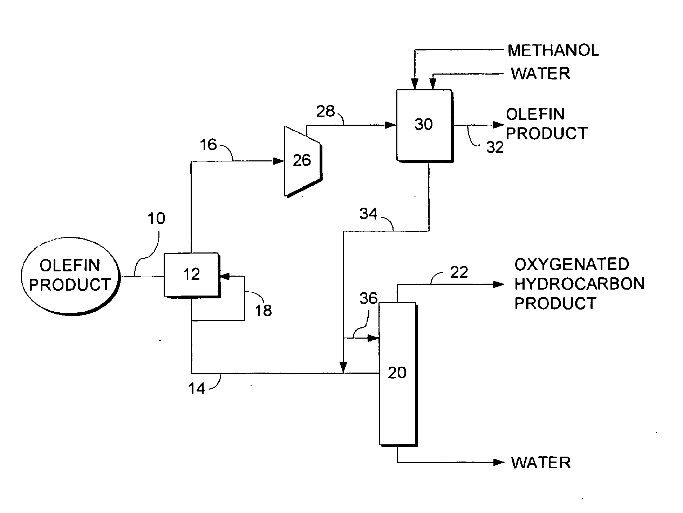

### BRIEF DESCRIPTION OF THE DRAWING

One embodiment of invention is shown in the attached Figure, which is a flow diagram showing one particular embodiment, for separating and recovering oxygenated hydrocarbon from the olefin product of an oxygenate to olefin reaction process.

### DETAILED DESCRIPTION OF THE INVENTION

This invention provides a method of recovering oxygenated hydrocarbon from an olefin stream produced in an oxygenate to olefin pmcess. In an oxygenate to olefin porcess, an oxygenate feed stream, typically a methanol or a methanol blend, is converted into an olefin stream. The olefin stream contains significant amounts of ethylene and propylene, as well as a significant amount of water, which is a normal by-product in the catalytic conversion of methanol to olefin. The olefin stream also contains various amounts of oxygenated hydrocarbon by-products, which are produced as a result of incomplete conversion or undesirable side reactions. A significant amount of the oxygenated hydrocarbon by-products is recovered by the method of this invention.

An olefin stream is obtained by contacting oxygenate with a molecular sieve catalyst. The oxygenate comprises at least one organic compound which contains at least one oxygen atom, such as aliphatic alcohols, ethers, carbonyl compounds (aldehydes, ketones, carboxylic acids, carbonates, esters and the like). When the oxygenate is an alcohol, the alcohol includes an aliphatic moiety having from 1 to 10 carbon atoms, more preferably from 1 to 4 carbon atoms. Representative alcohols include but are not necessarily limited to lower straight and branched chain aliphatic alcohols and their unsaturated counterparts. Examples of suitable oxygenate compounds include, but are not limited to: methanol; ethanol; n-propanol; isopropanol; C₄- C₂₀ alcohols; methyl ethyl ether; dimethyl ether; diethyl ether; di-isopropyl ether; formaldehyde; dimethyl carbonate; dimethyl ketone; acetic acid; and mixtures thereof. Preferred oxygenate compounds are methanol, dimethyl ether, or a mixture thereof.

The molecular sieve catalyst used in this invention is an oxygenate to olefin catalyst, which is defined as any molecular sieve capable of converting an oxygenate to an olefin compound. Such molecular sieves include zeolites as well as non-zeolites, and are of the large, medium or small pore type. Small pore molecular sieves are preferred in one embodiment of this invention, however. As defined herein, small pore molecular sieves have a pore size of less than 5.0.10⁻¹⁰ m (5.0 angstroms). Generally, suitable catalysts have a pore size ranging from 3.5 to 5.0 angstroms, preferably from 4.0.10⁻¹⁰m 5.0.10⁻¹⁰m (4.0 to 5.0 angstorms), and most preferably from 4.3.10⁻¹⁰m to 5.0.10⁻¹⁰m. (4.3 to 5.0 angstorms).

Zeolite materials, both natural and synthetic, have been demonstrated to have catalytic properties for various types of hydrocarbon conversion processes. In addition, zeolite materials have been used as adsorbents, catalyst carriers for various types of hydrocarbon conversion processes, and other applications. Zeolites are complex crystalline aluminosilicates which form a network of AlO₂⁻ and SiO₂ tetrahedra linked by shared oxygen atoms. The negativity of the tetrahedra is balanced by the inclusion of cations such as alkali or alkaline earth metal ions. In the manufacture of some zeolites, non-metallic cations, such as tetramethylammonium (TMA) or tetrapropylammonium (TPA), are present during synthesis. The interstitial spaces or channels formed by the crystalline network enable zeolites to be used as molecular sieves in separation processes, as catalyst for chemical reactions, and as catalyst carriers in a wide variety of hydrocarbon conversion processes.

Zeolites include materials containing silica and optionally alumina, and materials in which the silica and alumina portions have been replaced in whole or in part with other oxides. For example, germanium oxide, tin oxide, and mixtures thereof can replace the silica portion. Boron oxide, iron oxide, gallium oxide, indium oxide, and mixtures thereof can replace the alumina portion. Unless otherwise specified, the terms "zeolite" and "zeolite material" as used herein, shall mean not only materials containing silicon atoms and, optionally, aluminum atoms in the crystalline lattice structure thereof, but also materials which contain suitable replacement atoms for such silicon and aluminum atoms.

One preferred type of olefin forming catalyst useful in this invention is one containing a silicoaluminophosphate (SAPO) molecular sieve. Silicoaluminophosphate molecular sieves are generally classified as being microporous materials having 8, 10, or 12 membered ring structures. These ring structures can have an average pore size ranging from 3.5.10⁻¹⁰ to 15.10⁻¹⁰ (3.5 to 15 angstroms). Preferred are the small pore SAPO molecular sieves havin an avera ge pore size of less than 5.10⁻¹⁰m (5 angstroms), preferably an average pore size ranging from 3.5.10⁻¹⁰ to 5.10⁻¹⁰ m (3.5 to 5 angstroms), more preferably form 3.5.10⁻¹⁰ to 4.2.10⁻¹⁰m (3.5 to 4.2 angstroms). These pore sizes are typical of molecular sieves having 8 membered rings.

According to one embodiment, substituted SAPOs can also be used in oxygenate to olefin reaction processes. These compounds are generally known as MeAPSOs or metal-containing silicoaluminophosphates. The metal can be alkali metal ions (Group IA), alkaline earth metal ions (Group IIA), rare earth ions (Group IIIB, including the lanthanoid elements: lanthanum, cerium, praseodymium, neodymium, samarium, europium, gadolinium, terbium, dysprosium, holmium, erbium, thulium, ytterbium and lutetium; and scandium or yttrium) and the additional transition cations of Groups IVB, VB, VIB, VIIB, VIIIB, and IB.

Preferably, the Me represents atoms such as Zn, Mg, Mn, Co, Ni, Ga, Fe, Ti, Zr, Ge, Sn, and Cr. These atoms can be inserted into the tetrahedral framework through a [MeO₂] tetrahedral unit. The [MeO₂] tetrahedral unit carries a net electric charge depending on the valence state of the metal substituent. When the metal component has a valence state of +2, +3, +4, +5, or +6, the net electric charge is between -2 and +2. Incorporation of the metal component is typically accomplished adding the metal component during synthesis of the molecular sieve. However, post-synthesis ion exchange can also be used. In post synthesis exchange, the metal component will introduce cations into ion-exchange positions at an open surface of the molecular sieve, not into the framework itself.

Suitable silicoaluminophosphate molecular sieves include SAPO-5, SAPO-8, SAPO-11, SAPO-16, SAPO-17, Saps- 18 SAPO-20, SAPO-31, SAPO-34, SAPO-35, SAPO-36, SAPO-37, SAPO-40, SAPO-41, SAPO-42, SAPO-44, SAPO-47, SAPO-56, the metal containing forms thereof, and mixtures thereof. Preferred are SAPO-18, SAPO-34, SAPO-35, SAPO-44, and SAPO-47, particularly SAPO-18 and SAPO-34, including the metal containing forms thereof, and mixtures thereof. As used herein, the term mixture is synonymous with combination and is considered a composition of matter having two or more components in varying proportions, regardless of their physical state.

An aluminophosphate (ALPO) molecular sieve can also be included in the catalyst composition. Aluminophosphate molecular sieves are crystalline microporous oxides which can have an AlPO₄ framework. They can have additional elements within the framework, typically have uniform pore dimensions ranging from 3.10⁻¹⁰m (3 angstroms) to 10⁻⁹m (10 angstroms), and are capable of making size selective separations of molecular species. More than two dozen structure types have been reported, including zeolite topological analogues. A more detailed description of the background and synthesis of aluminophosphates is found in U.S. Pat. No. 4,310,440. Preferred ALPO structures are ALPO-5, ALPO-11, ALPO-18, ALPO-31, ALPO-34, ALPO-36, ALPO-37, and ALPO-46.

The ALPOs can also include a metal substituent in its framework. Preferably, the metal is selected from the group consisting of magnesium, manganese, zinc, cobalt, and mixtures thereof. These materials preferably exhibit adsorption, ion-exchange and/or catalytic properties similar to aluminosilicate, aluminophosphate and silica aluminophosphate molecular sieve compositions. Members of this class and their preparation are described in U.S. Pat. No. 4,567,029.

The metal containing ALPOs have a three-dimensional microporous crystal framework structure of MO₂, AlO₂ and PO₂ tetrahedral units. These as manufactured structures (which contain template prior to calcination) can be represented by empirical chemical composition, on an anhydrous basis, as:

mR: (MₓAl_{y}P_{z})O₂

wherein "R" represents at least one organic templating agent present in the intracrystalline pore system; "m" represents the moles of "R" present per mole of (MₓAl_{y}P_{z})O₂ and has a value of from zero to 0.3, the maximum value in each case depending upon the molecular dimensions of the templating agent and the available void volume of the pore system of the particular metal aluminophosphate involved, "x", "y", and "z" represent the mole fractions of the metal "M", (i.e. magnesium, manganese, zinc and cobalt), aluminum and phosphorus, respectively, present as tetrahedral oxides.

The metal containing ALPOs are sometimes referred to by the acronym as MeAPO. Also in those cases where the metal "Me" in the composition is magnesium, the acronym MAPO is applied to the composition. Similarly ZAPO, MnAPO and CoAPO are applied to the compositions which contain zinc, manganese and cobalt respectively. To identify the various structural species which make up each of the subgeneric classes MAPO, ZAPO, CoAPO and MnAPO, each species is assigned a number and is identified, for example, as ZAPO-5, MAPO-11, CoAPO-34 and so forth.

The silicoaluminophosphate molecular sieve is typically admixed (i.e., blended) with other materials. When blended, the resulting composition is typically referred to as a SAPO catalyst, with the catalyst comprising the SAPO molecular sieve.

Materials which can be blended with the molecular sieve can be various inert or catalytically active materials, or various binder materials. These materials include compositions such as kaolin and other clays, various forms of rare earth metals, metal oxides, other non-zeolite catalyst components, zeolite catalyst components, alumina or alumina sol, titania, zirconia, magnesia, thoria, beryllia, quartz, silica or silica or silica sol, and mixtures thereof. These components are also effective in reducing, inter alia, overall catalyst cost, acting as a thermal sink to assist in heat shielding the catalyst during regeneration, densifying the catalyst and increasing catalyst strength. It is particularly desirable that the inert materials that are used in the catalyst to act as a thermal sink have a heat capacity of from 0.05 to 1 cal/g-°C, more preferably from 0.1 to 0.8 cal/g-°C, most preferably from 0.1 to 0.5 cal/g-°C.

Additional molecular sieve materials can be included as a part of the SAPO catalyst composition or they can be used as separate molecular sieve catalysts in admixture with the SAPO catalyst if desired. Structural types of small pore molecular sieves that are suitable for use in this invention include AEI, AFT, APC, ATN, ATT, ATV, AWW, BIK, CAS, CHA, CHI, DAC, DDR, EDI, ERI, GOO, KFI, LEV, LOV, LTA, MON, PAU, PHI, RHO, ROG, THO, and substituted forms thereof. Structural types of medium pore molecular sieves that are suitable for use in this invention include MFI, MEL, MTW, EUO, MTT, HEU, FER, AFO, AEL, TON, and substituted forms thereof. These small and medium pore molecular sieves are described in greater detail in the Atlas of Zeolite Structural Types, W.M. Meier and D.H. Olsen, Butterworth Heineman, 3rd ed., 1997. Preferred molecular sieves which can be combined with a silicoaluminophosphate catalyst include ZSM-5, ZSM-34, erionite, and chabazite.

The catalyst composition, according to an embodiment, preferably comprises from 1% to 99 %, more preferably from 5 % to 90 %, and most preferably from 10% to 80%, by weight of molecular sieve. It is also preferred that the catalyst composition have a particle size of from 20 angstroms to 3,000 angstroms, more preferably from 3.10⁻⁹ m (30 angstroms) to 2.10⁻⁸ m (200 angstroms), most preferably from 5.10⁻⁹ m (50 angstroms) to 15.10⁻⁹ m (150 angstroms).

The catalyst can be subjected to a variety of treatments to achieve the desired physical and chemical characteristics. Such treatments include, but are not necessarily limited to hydrothermal treatment, calcination, acid treatment, base treatment, milling, ball milling, grinding, spray drying, and combinations thereof.

A preferred catalyst of this invention is a catalyst which contains a combination of SAPO-34, and SAPO-18 or ALPO-18 molecular sieve. In a particularly preferred embodiment, the molecular sieve is a crystalline intergrowth of SAPO-34, and SAPO-18 or ALPO-18.

To convert oxygenate to olefin, conventional reactor systems can be used, including fixed bed, fluid bed or moving bed systems. Preferred reactors of one embodiment are co-current riser reactors and short contact time, countercurrent free-fall reactors. Desirably, the reactor is one in which an oxygenate feedstock can be contacted with a molecular sieve catalyst at a weight hourly space velocity (WHSV) of at least 1 hr-¹, preferably in the range of from 1 hr⁻¹ to 1000 hr⁻¹, more preferably in the range of from 20 hr⁻¹ to 1000 hr⁻¹, and most preferably in the range of from 50 hr⁻¹ to 500 hr⁻¹. WHSV is defined herein as the weight of oxygenate, and reactive hydrocarbon which may optionally be in the feed, per hour per weight of the molecular sieve in the reactor. Because the catalyst or the feedstock may contain other materials which act as inerts or diluents, the WHSV is calculated on the weight basis of the oxygenate feed, and any reactive hydrocarbon which may be present with the oxygenate feed, and the molecular sieve contained in the reactor.

Preferably, the oxygenate feed is contacted with the catalyst when the oxygenate is in a vapor phase. Alternately, the process may be carried out in a liquid or a mixed vapor/liquid phase. When the process is carried out in a liquid phase or a mixed vapor/liquid phase, different conversions and selectivities of feed-to-product may result depending upon the catalyst and reaction conditions.

The process can generally be carried out at a wide range of temperatures. An effective operating temperature range can be from 200°C to 700°C, preferably from 300°C to 600°C, more preferably from 350°C to 550°C. At the lower end of the temperature range, the formation of the desired olefin products may become markedly slow with a relatively high content of oxygenated olefin by-products being found in the olefin product. However, the selectivity to ethylene and propylene at reduced temperatures may be increased. At the upper end of the temperature range, the process may not form an optimum amount of ethylene and propylene product, but the conversion of oxygenate feed will generally be high.

The pressure also may vary over a wide range, including autogenous pressures. Effective pressures include, but are not necessarily limited to, oxygenate partial pressures of at least 1 psia (7 kPa), preferably at least 5 psia (34 kPa). The process is particularly effective at higher oxygenate partial pressures, such as an oxygenate partial pressure of greater than 20 psia (138 kPa). Preferably, the oxygenate partial pressure is at least 25 psia (172 kPa), more preferably at least 30 psia (207 kPa). For practical design purposes it is desirable to operate using methanol feed at a partial pressure of not greater than 500 psia (3445 kPa), preferably not greater than 400 psia (2756 kPa), most preferably not greater than 300 psia (2067 kPa).

One important conversion process condition according to one embodiment of the invention is gas superficial velocity. As the gas superficial velocity increases the conversion decreases avoiding undesirable by-products. As used herein, the term, "gas superficial velocity" is defined as the combined volumetric flow rate of vaporized feedstock, which includes diluent when present in the feedstock, as well as conversion products, divided by the cross-sectional area of the reaction zone. Because the oxygenate is converted to a product having significant quantities of ethylene and propylene while flowing through the reaction zone, the gas superficial velocity may vary at different locations within the reaction zone. The degree of variation depends on the total number of moles of gas present and the cross section of a particular location in the reaction zone, temperature, pressure and other relevant reaction parameters.

In one embodiment, the gas superficial velocity is maintained at a rate of greater than 1 meter per second (m/s) at least one point in the reaction zone. In another embodiment, it is desirable that the gas superficial velocity is greater than 2 m/s at least one point in the reaction zone. More desirably, the gas superficial velocity is greater than 2.5 m/s at least one point in the reaction zone. Even more desirably, the gas superficial velocity is greater than 4 m/s at least one point in the reaction zone. Most desirably, the gas superficial velocity is greater than 8 m/s at least one point in the reaction zone.

According to yet another embodiment of the invention, the gas superficial velocity is maintained relatively constant in the reaction zone such that the gas superficial velocity is maintained at a rate greater than 1 m/s at all points in the reaction zone. It is also desirable that the gas superficial velocity be greater than 2 m/s at all points in the reaction zone. More desirably, the gas superficial velocity is greater than 2.5 m/s at all points in the reaction zone. Even more desirably, the gas superficial velocity is greater than 4 m/s at all points in the reaction zone. Most desirably, the gas superficial velocity is greater than 8 m/s at all points in the reaction zone.

The amount of ethylene and propylene produced in the oxygenate to olefin process can be increased by reducing the conversion of the oxygenates in the oxygenate to olefins reaction. This is because a high conversion of feed oxygenates tends to form additional undesirable non-olefin by-products. However, reducing the conversion of feed oxygenates in the oxygenate conversion reaction tends to increase the amount of oxygenated hydrocarbons that are present in the olefin product. Thus, control of the conversion of feed to the oxygenate reaction process can be important.

According to one embodiment, the conversion of the primary oxygenate, e.g., methanol, is from 90 wt % to 98 wt %. According to another embodiment the conversion of methanol is from 92 wt % to 98 wt %, preferably from 94 wt % to 98 wt %.

According to another embodiment, the conversion of methanol is above 98 wt % to less than 100 wt %. According to another embodiment, the conversion of methanol is from 98.1 wt % to less than 100 wt %; preferably from 98.2 wt % to 99.8 wt %. According to another embodiment, the conversion of methanol is from 98.2 wt % to less than 99.5 wt %; preferably from 98.2 wt % to 99 wt %.

In this invention, weight percent conversion is calculated on a water free basis unless otherwise specified. Weight percent conversion on a water free basis is calculated as: 100 x (weight oxygenate fed on a water free basis - weight oxygenated hydrocarbon in the product on a water free basis). The water free basis of oxygenate is calculated by subtracting out the water portion of the oxygenate in the feed and product, and excluding water formed in the product. For example, the weight flow rate of methanol on an oxygenate free basis is calculated by multiplying the weight flow rate of methanol by 14/32 to remove the water component of the methanol. As another example, the rate flow rate of dimethylether on an oxygenate free basis is calculated by multiplying the weight flow rate of diemethylether by 40/46 to remove the water component of the dimethylether. If there is a mixture of oxygenates in the feed or product, trace oxygenates are not included. When methanol and/or dimethylether is used as the feed, only methanol and dimethylether are used to calculate conversion on a water free basis.

In this invention, selectivity is also calculated on a water free basis unless otherwise specified. Selectivity is calculated as: 100 x wt % component / (100 - wt % water - wt % methanol - wt % dimethylether) when methanol and/or dimethylether is used as the feed.

Desirably, the amount of oxygenated hydrocarbon present in the olefin stream from the oxygenate to olefm reaction, on a water free basis, is at least 100 ppm by weight, preferably at least 500 ppm by weight, and more preferably at least 1000 ppm by weight. The amount of oxygenated hydrocarbon in the olefin stream, on an water free basis, should not be so high, however, to significantly affect removal of the oxygenated hydrocarbon from the desirable olefin product. Preferably, the amount of oxygenated hydrocarbon in the olefin stream from the oxygenate to olefm reaction process should be not greater than 2 wt %, more preferably not greater than 1 wt %, and most preferably not greater than 0.5 wt %.

The olefin stream from the oxygenate conversion reaction generally contains a variety of hydrocarbon and non-hydrocarbon components, including water. Typically, the olefin stream will contain at least 40 wt % water and at least 30 wt % hydrocarbons. On a water containing basis, the olefin stream desirably has less than 25 wt % oxygenated hydrocarbons, preferably less than 20 wt %, and more preferably less than 10 wt %. Preferably, on a water containing basis, at least 20 wt % of the olefin stream comprises ethylene and propylene. The ratio of ethylene to propylene can be adjusted as desired by changing catalyst and reactor conditions. Other non-oxygenated hydrocarbons in the product stream can include C₁ to C₇ paraffins, C₄ to C₇ olefins, and a variety of other saturated and unsaturated hydrocarbons.

The olefin stream will also contain a variety of oxygenated hydrocarbon components. These components include, for example, methanol, ethanol, C₃ alcohols, dimethyl ether, methyl ether, C₄ ethers, acetic acid, propanoic acid, butyric acid, C₂ to C₆ aldehydes, and C₃ to C₆ ketones.

The amount of oxygenated hydrocarbons in the olefin stream will vary with the degree of oxygenate feed conversion. At lower oxygenate conversion levels, there is an increase in the amount of oxygenated hydrocarbons in the product stream. According to one embodiment, at least 0.5 wt % of the olefin stream, on a water containing basis, comprises oxygenated hydrocarbons. Another embodiment comprises, on a water containing basis, at least 1 wt %, and yet another embodiment, on a water containing basis, comprises at least 2.5 wt %.

A significant amount of water in the olefin stream from the oxygenate to olefin reaction process is removed by cooling the stream to a temperature below the condensation temperature of the water vapor in the stream. Preferably, the temperature of the product stream is cooled to a temperature below the condensation temperature of the oxygenate feed. In certain embodiments it is desirable to cool the product stream below the condensation temperature of methanol.

In one embodiment, the olefm stream from the oxygenate to olefin reaction process is cooled, then the cooled olefin stream is separated into two fractions. One fraction is a condensed, water containing stream which comprises most of the water from the olefin stream and a significant portion of the oxygenated hydrocarbons from the olefin stream. Another fraction is an olefin vapor stream which comprises a majority of the olefins, e.g., ethylene and propylene.

The olefin stream from the oxygenate to olefin reaction process is cooled so that the cooled, condensed water containing stream contains at least 1 wt % oxygenated hydrocarbon, preferably at least 2 wt % oxygenated hydrocarbon, more preferably at least 3 wt % oxygenated hydrocarbon. It is preferred that the cooled, condensed water containing stream contain less than 1000 ppm by weight olefin components, preferably less than 500 ppm olefin components, more preferably less than 250 ppm olefin.

In another embodiment, the olefin stream from the oxygenate to olefin reaction process is cooled so that a vapor stream, rich in olefins, is separated from the condensed water containing stream. It is preferred that the vapor stream contain not greater than 20 wt % water, preferably not greater than 15 wt % water, more preferably not greater than 12 wt % water.

In one embodiment, the vapor stream contains not greater than 15 wt % oxygenated hydrocarbon, preferably not greater than 12 wt % oxygenated hydrocarbon, more preferably not greater than 10 wt % oxygenated hydrocarbon. In one embodiment, the vapor stream contains not greater than 15 wt % methanol and dimethylether, preferably not greater than 12 wt % methanol and dimethylether, and more preferably not greater than 10 wt % methanol and dimethylether.

In one embodiment of the invention, the olefin stream from the olefin to oxygenate reaction process is cooled at a pressure range that is not greater than that at which the oxygenate to olefin reaction process is carried out. Preferably, the olefin stream is cooled at a pressure of not greater than 50 psia (345 kPa), more preferably not greater than 40 psia (276 kPa).

A quench column is one type of equipment that is effective in cooling the olefin stream from the olefin to oxygenate reaction process. In a quench column, a quenching fluid is directly contacted with the olefin stream to cool the stream to the desired condensation temperature. Condensation produces the condensed water containing stream, which is also referred to as a heavy bottoms stream. The olefin portion of the olefin product stream remains a vapor, and exits the quench column as an overhead vapor stream. The overhead vapor stream is rich in olefin product, and can also contain some oxygenated hydrocarbon by-products.

In one embodiment, the quenching fluid is a recycle stream of the condensed water containing, heavy bottoms stream of the quench column. This water containing stream is desirably cooled, e.g., by a heat exchanger, and injected back into the quench column. It is preferred in this embodiment to not inject cooling medium from an outside source into the quench column, although it may be desirable to do so in other separation equipment down stream of the quench column.

In one embodiment, the oxygenated hydrocarbon is further separated from the cooled, condensed water containing stream. Conventional separation processes can be used, distillation being one example of a separation process. The separated oxygenated hydrocarbon can then be used as additional feed for the oxygenate reaction or it can be used as fuel or for other processing.

The oxygenated hydrocarbon stream separated from the cooled, condensed water stream should be low in water. In one embodiment the separated oxygenated hydrocarbon stream contains not greater than 50 wt % water. Lower concentrations of water in the separated stream with concentrations of not greater than 40 wt %, 30 wt %, and 25 wt % being increasingly preferred.

The oxygenated hydrocarbon stream that is separated from the cooled, condensed water containing stream should also contain a relatively high percentage of oxygenated hydrocarbons. Desirably, the separated stream contains at least 50 wt % of the oxygenated hydrocarbons present in the olefin stream from the oxygenate to olefin reaction process. Higher proportions of oxygenated hydrocarbons extracted from the olefm product stream are preferred. Separated streams containing at least 60 wt %, at least 70 wt % and at least 80 wt % of the oxygenated hydrocarbons present in the olefin stream from the oxygenate reaction process are increasingly preferred.

This invention provides for additional oxygenated hydrocarbon recovery by compressing the vapor stream formed from cooling the olefin stream from the oxygenate to olefin reaction process. Compressing the vapor stream condenses water and various oxygenated hydrocarbon compounds which were not condensed by merely cooling the olefm stream. These additionally condensed compounds are combined with the condensed water containing stream and the oxygenated hydrocarbon is separated and recovered. Optionally, the compressed condensate stream can be methanol and/or water washed and oxygenated hydrocarbon separated.

In one embodiment of the invention, the vapor stream is compressed to a pressure that is greater than that at which the oxygenate to olefin reaction process is carried out. Preferably, the vapor stream is compressed to a pressure of at least 30 psia (207 kPa), more preferably at least 50 psia (345 kPa), most preferably at least 100 psia (689 kPa). High pressure ranges are particularly preferred, with the upper limit being a practical one based on cost of design and ease of operation. Practical high pressure limits are generally considered to be up to 5,000 psia (34,450 kPa), with lower limits of 1,000 psia (6,895 kPa), 750 psia (5171 kPa), and 500 psia (3447 kPa) being increasingly preferred.

Yet another embodiment of this invention provides for additional oxygenated hydrocarbon recovery. This additional recovery results by methanol and/or water washing the vapor stream formed from cooling the olefin stream from the oxygenate to olefin reaction process. The methanol and/or water washed product stream is then combined with the condensed water containing stream, and the oxygenated hydrocarbon is separated as described above.

Desired temperature ranges for methanol and/or water washing the vapor stream are from 40°F (4°C) to 200°F (93°C), preferably from 60°F (16°C) to 150°F (66°C), more preferably from 80°F (27°C) to 120°F (49°C). A desired flow rate of methanol and/or water is 0.01 to 20.0 times the flow rate of olefm vapor stream, which can be either before or after optional compression. Other desired flow ranges include 0.05 to 10.0 times the flow rate of olefin vapor stream; 0.05 to 5.0 times the flow rate of olefm vapor stream; and 0.05 to 2.0 times the flow rate of olefin vapor stream. It is also beneficial to maintain the wash rate so that the liquid product of the wash contain not greater than 10 wt % of olefin entering the wash unit. It is increasing desirable to maintain the wash rate so that the liquid product of the wash contain not greater than 1.0 wt %, preferably not greater than 0.5 wt %, more preferably not greater than 0.1 wt %, and most preferably not greater than 0.01 wt % of the olefin entering the wash unit.

Conventional contacting units can be used to water and/or methanol wash the vapor stream. Columns with trays and/or packing are preferred wash units.

The oxygenated hydrocarbon product that is separated and recovered according to this invention comprises a mixture of oxygenates, including at least part of the unreacted oxygenate feedstock. One mixture of oxygenated hydrocarbons in the oxygenated hydrocarbon product of this invention is a composition which comprises not greater than 50 wt %, preferably not greater than 40 wt %, more preferably not greater than 30 wt % water, at least 25 wt %, preferably at least 30 wt %, more preferably at least 40 wt % of (a), wherein:
(a) is a compound of Formula I

   R-OH Formula I

   wherein R is C₁ to C₅ alkyl;
   and from 10 ppm by weight, preferably from 500 ppm by weight, more preferably from 1,000 ppm by weight to 10 wt %, preferably 8 wt %, more preferably 6 wt %, of at least two compounds selected from the group consisting of (b), (c), (d), or a combination thereof, wherein:
(b) is a compound of Formula II

   R₁-O-R₂ Formula II

   wherein R₁ is C₁ to C₄ alkyl and R₂ is C₁ to C₄ alkyl, wherein R₁ may be the same as or different than R₂;
(c) is a compound of Formula III 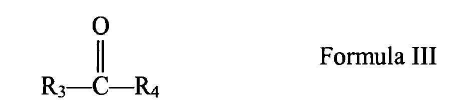 wherein R₃ is C₁ to C₃ alkyl and R₄ is C₁ to C₃ alkyl, wherein R₃ may be the same as or different than R₄; and
(d) is a compound of Formula IV 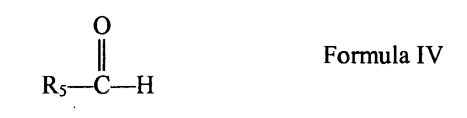 wherein R₅ is C₁ to C₅ alkyl.

The mixture of oxygenated hydrocarbons in the oxygenated hydrocarbon product of this invention optionally comprises from 5 ppm by weight, preferably from 250 ppm by weight, more preferably from 500 ppm by weight to 5 wt %, preferably 3 wt %, more preferably 2 wt %, of (e), (f), or a combination thereof, wherein:
(e) is a compound of Formula V 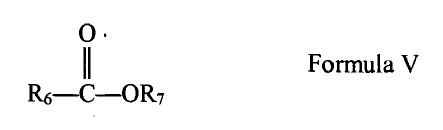 wherein R₆ is C₁ to C₃ alkyl and R₇ is C₁ to C₃ alkyl, wherein R₆ may be the same as or different than R₇; and
(f) is a compound of Formula VI 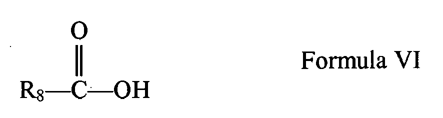 wherein R₈ is C₁ to C₅ alkyl.

Representative compounds of Formula I include methanol, ethanol, 1-propanol, 2-propanol, C₄ and C₅ alcohols. Representative compounds of Formula II include dimethyl ether, methyl ethyl ether, and C₄ ethers. Representative compounds of Formula III include acetone, C₄ ketones, C₅ ketones and C₆ ketones. Representative compounds of Formula IV include acetaldehyde, C₃ aldehydes, C₄ aldehydes, and C₅ aldehydes. Representative compounds of Formula V include C₃ esters, C₄ esters, and C₅ esters. Representative compounds of Formula VI include C₂ acids, C₃ acids, and C₄ acids.

The compounds of Formula I are present relative to the total amount of the at least two compounds of Formulas II, III and IV, in the separated oxygenated hydrocarbon, preferably at a weight ratio of at least 2:1, preferably at least 3:1, more preferably at least 5:1. When compounds of Formulas V and VI are included in the separated hydrocarbon, the compounds of Formula I will be present at a weight ratio of at least 5:1, preferably at least 7:1, more preferably at least 10:1, relative to the combination of the compounds of Formulas V and VI.

Preferably the separated oxygenated hydrocarbon is a composition that has at least 15 wt % methanol, at least 10 ppm by weight dimethylether, at least 10 ppm by weight acetone, butanone, acetaldehyde, propanal, butanal or a combination thereof, and not greater than 50 wt % water. More preferably, the separated oxygenated hydrocarbon is a composition that has at least 20 wt % methanol, from 10 ppm by weight to 10 wt % dimethylether, from 10 ppm by weight to 10 wt % propanal, from 10 ppm by weight to 10 wt % butanone, and not greater than 50 wt % water.

The separated oxygenated hydrocarbon provides an oxygenate product stream that can be used directly as an oxygenated hydrocarbon source for further chemical conversion or as a fuel. One desirable use of the separated oxygenated hydrocarbon is as a co-feed to the oxygenate to olefin reactor. The separated oxygenated hydrocarbon can be fed directly to the reactor, or preferably, mixed with another oxygenated hydrocarbons, like methanol, and then fed to the reactor. Another use of the separated oxygenated hydrocarbon is as a fuel. The separated oxygenated hydrocarbon can be combusted, and heat energy can be recovered and used as a heat source in the oxygenate to olefin process or other processes.

A general embodiment of this invention is shown in the Figure. In the Figure, olefin product from an oxygenate to olefins reaction is sent through a line 10 to a cooling unit, e.g., quench tower, 12. Cooled olefin product is separated as an olefin vapor stream, exiting cooling unit 12 through a line 16, and a water containing stream is removed from cooling unit 12 through a line 14. The cooling unit is kept cool by recycling a portion of the water containing stream through a line 18. The line 18 preferably includes a heat exchange device (not shown) to cool the recycled stream.

The water containing stream is sent to a separator 20, preferably a distillation column. Separator 20 is used to separate oxygenated hydrocarbon from the water containing stream. The oxygenated hydrocarbon is removed through a line 22, with the remaining water being removed through a line 24.

The olefin vapor stream is sent through the line 16 and, optionally, through a compressor 26. Compressed olefin is sent through a line 28 and to a separator 30 where a liquid oxygenated hydrocarbon containing stream, is removed through a line 34. Olefin vapor product is removed from the separator through a line 32.

If desired, methanol and/or water washes can be used to increase the amount of oxygenated hydrocarbon that is removed through the line 34. Desirably such washes are carried out in different separator vessels, and operated either in parallel or in series.

Oxygenated hydrocarbons removed through the line 34 are sent to separator 20 to further concentrate the oxygenated hydrocarbon. The oxygenated hydrocarbons removed through the line 34 can be directly combined with the condensed water containing stream in line 14 or they can be sent directly to the separator 20 through a line 36. The result is that an oxygenated hydrocarbon product can be recovered from the separator 20 that is reduced in water content and can be used as co-deed for the oxygenate reaction process or used as fuel.

## Claims

1. A process for separating oxygenated hydrocarbon from an olefin composition containing at least 40 wt% water and at least 30 wt% hydrocarbons, comprising:
a) contacting an oxygenate with a molecular sieve catalyst to form an olefin composition, wherein the olefin composition comprises olefins, at least 40 wt% water and less than 25wt% oxygenated hydrocarbon, said olefin stream comprising at least 20 wt% of ethylene and propylene and at least 0.5 wt% of oxygenated hydrocarbons;
b) cooling the olefin composition to form a liquid water containing stream and an olefin containing vapor stream, wherein the water containing stream comprises at least 1 wt % oxygenated hydrocarbon;
c) separating the water containing stream from the vapor stream;
d) compressing the vapor stream;
e) separating an olefin product stream and an oxygenated hydrocarbon containing stream from the compressed vapor stream;
f) combining the water containing stream and the oxygenated hydrocarbon containing stream; and
g) recovering an oxygenated hydrocarbon product from the combined water containing stream and liquid oxygenated hydrocarbon containing stream.

2. The process of claim 1, further comprising separating the olefin product into an ethylene containing stream and a propylene containing stream.

3. The process of claim 2, further comprising polymerizing the ethylene containing stream.

4. The process of claim 2, further comprising polymerizing the propylene containing stream.

5. The process of claim 1, wherein the water containing stream and the oxygenated hydrocarbon containing stream are first combined and then separated in a separator.

6. The process of claim 1, wherein the water containing stream and the oxygenated hydrocarbon containing stream are both combined and separated within a separator.

7. The process of claim 1, wherein the vapor stream is compressed at a pressure of at least 207 kPa.

## Patentansprüche

1. Verfahren zur Separierung eines oxygenierten Kohlenwasserstoffs von einer Olefinzusammensetzung, die mindestens 40 Gew.% Wasser und mindestens 30 Gew.% Kohlenwasserstoffe umfasst, bei dem:
a) ein Oxygenat mit einem Molekularsiebkatalysator zur Bildung einer Olefinzusammensetzung in Kontakt gebracht wird, wobei die Olefinzusammensetzung Olefine, mindestens 40 Gew.% Wasser und weniger als 25 Gew.% oxygenierte Kohlenwasserstoffe umfasst, wobei der Olefinstrom mindestens 20 Gew.% Ethylen und Propylen und mindestens 0,5 Gew.% oxygenierte Kohlenwasserstoffe umfasst;
b) die Olefinzusammensetzung zur Bildung einer Wasser enthaltenden Flüssigkeit und eines Olefin enthaltenden Dampfstroms abgekühlt wird, wobei der Wasser enthaltende Strom mindestens 1 Gew.% oxygenierten Kohlenwasserstoff enthält,
c) der Wasser enthaltende Strom vom Dampfstrom getrennt wird,
d) der Dampfstrom verdichtet wird,
e) ein Olefinproduktstrom und ein oxygenierte Kohlenwasserstoffe enthaltender Strom vom verdichteten Dampfstrom abgetrennt werden,
f) der Wasser enthaltende Strom und der oxygenierte Kohlenwasserstoffe enthaltende Strom kombiniert werden, und
g) ein oxygeniertes Kohlenwasserstoffprodukt aus dem kombinierten Wasser enthaltenden Strom und dem flüssige oxygenierte Kohlenwasserstoffe enthaltendem Strom gewonnen wird.

2. Verfahren nach Anspruch 1, bei dem weiterhin das Olefinprodukt in einen Ethylen enthaltenden Strom und einen Propylen enthaltenden Strom getrennt wird.

3. Verfahren nach Anspruch 2, bei dem weiterhin der Ethylen enthaltende Strom polymerisiert wird.

4. Verfahren nach Anspruch 2, bei dem weiterhin der Propylen enthaltende Strom polymerisiert wird.

5. Verfahren nach Anspruch 1, bei dem der Wasser enthaltende Strom und der oxygenierte Kohlenwasserstoffe enthaltende Strom zunächst kombiniert und anschließend in einem Separator getrennt werden.

6. Verfahren nach Anspruch 1, bei dem der Wasser enthaltende Strom und der oxygenierte Kohlenwasserstoffe enthaltende Strom zunächst kombiniert und anschließend innerhalb eines Separators getrennt werden.

7. Verfahren nach Anspruch 1, bei dem der Dampfstrom mit einem Druck von mindestens 207 kPa verdichtet wird.

## Revendications

1. Procédé de séparation d'un hydrocarbure oxygéné à partir d'une composition d'oléfines contenant au moins 40 % en poids d'eau et au moins 30 % en poids d'hydrocarbures, comprenant les étapes consistant à:
a) mettre en contact un composé oxygéné avec un catalyseur de type tamis moléculaire pour former une composition d'oléfines, la composition d'oléfines comprenant des oléfines, au moins 40 % en poids d'eau et moins de 25 % en poids d'hydrocarbure oxygéné, ledit courant d'oléfines comprenant au moins 20 % en poids d'éthylène et de propylène et au moins 0,5 % en poids d'hydrocarbures oxygénés;
b) refroidir la composition d'oléfines pour former un courant contenant de l'eau liquide et un courant de vapeur contenant des oléfines, le courant contenant de l'eau comprenant au moins 1 % en poids d'hydrocarbure oxygéné;
c) séparer le courant contenant de l'eau du courant de vapeur;
d) comprimer le courant de vapeur;
e) séparer un courant de produit oléfinique et un courant contenant de l'hydrocarbure oxygéné du courant de vapeur comprimé;
f) combiner le courant contenant de l'eau et le courant contenant de l'hydrocarbure oxygéné; et
g) récupérer un produit hydrocarbure oxygéné dans le courant contenant de l'eau et le courant de liquide contenant de l'hydrocarbure oxygéné combinés.

2. Procédé selon la revendication 1, comprenant en outre le fait de séparer le produit oléfinique en un courant contenant de l'éthylène et un courant contenant du propylène.

3. Procédé selon la revendication 2, comprenant en outre le fait de polymériser le courant contenant de l'éthylène.

4. Procédé selon la revendication 2, comprenant en outre le fait de polymériser le courant contenant du propylène.

5. Procédé selon la revendication 1, dans lequel le courant contenant de l'eau et le courant contenant de l'hydrocarbure oxygéné sont d'abord combinés et ensuite séparés dans un séparateur.

6. Procédé selon la revendication 1, dans lequel le courant contenant de l'eau et le courant contenant de l'hydrocarbure oxygéné sont à la fois combinés et séparés au sein d'un séparateur.

7. Procédé selon la revendication 1, dans lequel le courant de vapeur est comprimé à une pression d'au moins 207 kPa.
